# EUROPEAN PATENT SPECIFICATION

(11) **EP 4 389 056 B1**
(45) Date of publication and mention of the grant of the patent: **22.04.2026**
(21) Application number: 21967174.0
(22) Date of filing: 08.12.2021
(51) Int. Cl.: A61B 46/10, A61B 34/30

(54) **DRAPE**
ABDECKTUCH
CHAMP OPÉRATOIRE

(43) Date of publication of application: 26.06.2024
(73) Proprietor: RIVERFIELD Inc., Tokyo 107-0052 (JP)
(72) Inventor: MORITA, Naoya, Tokyo 160-0017 (JP)
(74) Representative: Adares PartGmbB
(86) International application number: PCT/JP2021/045105
(87) International publication number: WO 2023/105675

(56) References cited:
- WO-A1-2018/235151
- WO-A1-2021/199413
- JP-A- 2020 500 606
- JP-A- 2021 171 345
- US-A1- 2006 161 138
- US-A1- 2015 202 009
- US-A1- 2020 069 385

## Description

### TECHNICAL FIELD

The present invention relates to a drape that that isolates a medical robot and a surgical instrument from each other.

### BACKGROUND ART

Surgery using medical robots is attracting attention as a technique that enhances the possibility of not only reducing the burden on a surgeon but also the burden on a patient as well as the possibility of remote medical care through highly accurate and stable treatment. Surgical instruments that come into direct contact with patients are generally configured to be sterilizable in consideration of repeated uses, but the medical robot side provided with mechanisms to move surgical instruments is generally not sterilized because the electrical and electronic components on the robot side have no sterilization resistance. In the surgery using a medical robot, therefore, the medical robot side is covered with a drape that includes a soft and flexible sheet, and the interior (unclean area) covered with the drape and an area (clean area) in which the surgical instrument exposed from the drape is located are isolated from each other to maintain respective environments of the clean area and the unclean area.

Patent Document 1 discloses a surgical robot drape for covering a surgical robot arm that includes a drive device for providing drive to a surgical instrument. This drape includes a cover for covering the robotic arm and defining a sterile boundary thereon, and an interface element attached to the cover.

Patent Document 2 discloses a surgical robot drape for covering a part of a surgical robot. This drape includes a cover that covers a drive device of the surgical robot to define a sterile boundary thereon, and a plurality of interface elements.

Patent Document 3 discloses a bearing structure that, when there is a rotational operation of an insertion member, can reduce the possibility that the drape covering a rotating member, a fixation member, a holding body, an arm portion, etc. may be torn, and that can reliably maintain the isolation between the clean area and the unclean area.

Patent Document 4 discloses a surgical robot drape that covers a joint of the surgical robot. This drape has a proximal drape material portion and a distal drape material portion. The proximal drape material portion is fixed so as to form a sterile barrier covering a site proximal to the joint of the robot and rotate together with the site. The distal drape material portion is fixed so as to form a sterile barrier over a site distal to the joint of the robot and rotate together with the site.

Patent Document 5 describes a robot arm which is used in a clean area and is covered with a drape. An adaptor is attached to the robot arm while putting the drape between the adaptor and the robot arm, and a surgical instrument is attached to the adaptor.

Patent Document 6 describes a surgical instrument installed on an instrument sterile adaptor (ISA) integrated with a sterile drape covering a medical robot, wherein the ISA engages with an adapter receiving portion.

### PRIOR ART DOCUMENTS

### PATENT DOCUMENTS

Patent Document 1: Japanese translation of PCT international application, No. 2018-515212
Patent Document 2: Japanese translation of PCT international application, No. 2018-515213
Patent document 3: JP6806407B
Patent Document 4: Japanese translation of PCT international application, No. 2021-511876
Patent Document 5: US 2020/069385 A1.
Patent Document 6: US 2006/161138 A1.

### OBJECT AND SUMMARY OF THE INVENTION

Such a drape that isolates the clean area and the unclean area from each other may be provided with an attachment that transmits the operation of the drive mechanism of the medical robot to a member provided with the surgical instrument. When used, the attachment is attached to a predetermined position on the robot, but there is a concern that a flexible sheet continuous with the attachment may be pinched between the attachment and the robot during the attaching operation. If such a pinch occurs, it may cause poor fixation of the attachment or damage to the drape sheet.

An object of the present invention is to provide a drape that can avoid a pinch of the drape sheet when the drape is attached to the medical robot, thereby preventing poor fixation of the attachment and damage to the drape sheet.

To solve the above problem, an aspect of the present invention provides a drape that covers a medical robot, as defined in claim 1. Further embodiments of the invention are recited in the dependent claims. The drape comprises an attachment that is attached to a connection portion of the medical robot; a sheet having flexibility that is provided inseparably from the attachment and covers the medical robot; and a pinch prevention unit that is provided on the attachment and prevents the sheet from being pinched between the attachment and the connection portion.

By providing such a pinch prevention unit, it is possible to prevent the sheet having flexibility from being pinched between the attachment and the connection portion when covering the medical robot with the drape.

In the above drape, the flexibility of the pinch prevention unit is preferably higher than that of the attachment and lower than that of the sheet. By providing the pinch prevention unit with appropriate flexibility in this way, it is possible to prevent the sheet from coming too close to the attachment.

In the above drape, the pinch prevention unit preferably has translucency. This allows the attachment situation between the attachment and the connection portion to be referred to via the pinch prevention unit.

In the above drape, the pinch prevention unit preferably includes a plate-shaped body having an extending portion that extends outward from an outer surface of the attachment. Such a plate-shaped body can prevent the sheet from being pinched in the area outside the outer surface of the attachment.

In the above drape, the plate-shaped body is preferably inseparably attached to the sheet in an area including an end portion of the extending portion. This allows the clean area and the unclean area to be reliably isolated between the plate-shaped body and the sheet.

In the above drape, a first adhesive layer is provided on at least a part of ar outer surface of the pinch prevention unit, and the sheet and the pinch prevention unit are inseparably attached to each other by the first adhesive layer. Thus, the sheet and the pinch prevention unit are inseparably attached to each other by the first adhesive layer, and the clean area and the unclean area can thereby be reliably isolated between the sheet and the pinch prevention unit.

In the above drape, preferably, the pinch prevention unit includes a plate-shaped body having an extending portion that extends outward from an outer surface of the attachment, and the first adhesive layer is provided on one main surface of the plate-shaped body and extends to an end portion of the extending portion of the plate-shaped body. This allows the sheet and the plate-shaped body to be attached to each other by the first adhesive layer up to the end portion of the extending portion, and the clean area and the unclean area can be reliably isolated.

The above drape has a second adhesive layer provided on a side of the sheet opposite to a side to which the first adhesive layer is attached, and the sheet has an interposed portion that is interposed between the first adhesive layer and the second adhesive layer. Thus, the sheet is interposed between the first adhesive layer and the second adhesive layer by the interposed portion, and the clean area and the unclean area can thereby be reliably isolated.

In the above drape, the attachment is located on a side of the second adhesive layer opposite to a side facing the sheet. This allows the attachment to adhere by the second adhesive layer, and the clean area and the unclean area can be reliably isolated between the attachment and the sheet.

In the above drape, a cover film that protects the second adhesive layer is preferably provided on a side of the second adhesive layer opposite to a side facing the sheet. This can prevent the second adhesive layer from adhering to an unintended site at a stage before the sheet is adhered to the attachment.

In the above drape, the interposed portion may include a folded portion of the sheet. This allows the sheet including its folded portion to be interposed between the first adhesive layer and the second adhesive layer, and the clean area and the unclean area can be reliably isolated.

The above drape may cover a medical robot that includes a holder at an end of an arm and that detachably and rotatably holds an insertion member to be inserted into a patient. The attachment may have a bearing structure comprising: a ring-shaped fixation member that is attached with respect to the arm in a state in which the holder is inserted in the fixation member; and a ring-shaped rotating member that is slidably and rotatably attached with respect to the fixation member in a state in which the holder is inserted in the rotating member and that is rotatable together with the holder. The sheet may comprise: a first sheet that is attached to the fixation member and covers the arm; and a second sheet that is attached to the rotating member, covers the holder, and is rotatable together with the rotating member. With this configuration, in the sheet having the first sheet that covers the arm of the medical robot and the rotatable second sheet that covers the holder, it is possible to prevent jamming of the sheet when the drape is placed over the medical robot.

In the above drape, the pinch prevention unit is preferably provided on an outer periphery of the fixation member. With this configuration, when the first sheet is placed over the holder provided at the end of the arm of the medical robot, the pinch prevention unit can prevent jamming of the sheet on the outer periphery of the fixation member.

In the above drape, the pinch prevention unit is preferably composed of a plate-shaped body that extends in a direction away from the rotating member. Such a plate-shaped body can prevent jamming of the sheet in the area around the rotating member.

In the above drape, the rotating member preferably has a recessed portion on its outer periphery in which an adhesive layer is disposed. The adhesive layer allows the rotating member and the second sheet to be inseparably attached to each other. By disposing the adhesive layer in such a recessed portion, it is possible to reliably isolate the clean area and the unclean area from each other at the adhesion portion between the rotating member and the second sheet.

In the above drape, the attachment may have a separator that is disposed between the medical robot holding an insertion member to be inserted into a patient and the insertion member to isolate the insertion member and the medical robot from each other. The separator transmits power in a forward/backward direction from a power transmission part included in the medical robot to a movable part provided in the surgical instrument. The separator may comprise: a slider having a first engagement portion engaged with the power transmission part and a second engagement portion engaged with the movable part of the insertion member, the slider transmitting the power received from the power transmission part to the movable part; and a separator main body having a through-hole through which a part of the slider is inserted. The separator main body is detachably attached with respect to the medical robot. Thus, in the configuration having the separator as the attachment, when covering the medical robot with the drape, the pinch prevention unit can prevent the flexible sheet from being pinched between the attachment and the connection portion.

In the above drape, preferably, the drape has a first adhesive layer provided on a side of the pinch prevention unit facing the separator main body and a second adhesive layer provided on a side of the separator main body facing the pinch prevention unit, and the sheet has an interposed portion that is interposed between the first adhesive layer and the second adhesive layer. Thus, the sheet is interposed between the first adhesive layer and the second adhesive layer by the interposed portion, and the clean area and the unclean area can thereby be reliably isolated.

In the above drape, preferably, the pinch prevention unit has an opening portion, and the sheet is attached to the first adhesive layer so as not to cover the opening portion. With this configuration, the sheet is attached so as not to cover the through-hole of the pinch prevention unit.

In the above drape, the pinch prevention unit is preferably composed of a plate-shaped body. This allows the plate-shaped body to prevent the pinch of the sheet.

In the above drape, a portion of the plate-shaped body located around the slider preferably does not have a portion that protrudes further in a direction in a main surface of the plate-shaped body than the separator main body when the portion is viewed from a normal direction. With this configuration, the effect of preventing the sheet from being pinched can be obtained without extending the plate-shaped body more than necessary.

According to the present invention, it is possible to provide a drape that can avoid a pinch of the drape sheet when the drape is attached to the medical robot, thereby preventing poor fixation of the attachment and damage to the drape.

### BRIEF DESCRIPTION OF DRAWINGS

FIG. 1 is a perspective view for exemplifying the configuration of a medical robot;
FIG. 2 is a perspective view for exemplifying the attaching state of an insertion member;
FIG. 3 is a plan view for exemplifying a driving portion of the medical robot;
FIG. 4 is a cross-sectional view for exemplifying a connection configuration via a separator;
FIG. 5 is an exploded perspective view for exemplifying the schematic configuration of the arm tip side of the medical robot;
FIG. 6 is an exploded perspective view for exemplifying the configuration of a bearing structure;
FIGS. 7(a) and 7(b) are diagrams for exemplifying the structure of a drape;
FIG. 8 is a perspective view for exemplifying a pinch prevention unit;
FIGS. 9(a) and 9(b) are schematic cross-sectional views for exemplifying the pinch prevention unit;
FIG. 10 is a schematic cross-sectional view for exemplifying a connection portion between a second sheet and a rotating member;
FIG. 11 is a plan view for exemplifying an opening of the second sheet and the pinch prevention unit;
FIG. 12 is a perspective view for exemplifying a state of the second sheet and the separator before adhesion;
FIG. 13 is a perspective view for exemplifying a state of the second sheet and the separator after adhesion;
FIG. 14 is a schematic diagram for exemplifying coupling between the first sheet and the second sheet; and
FIG. 15 is a perspective view for exemplifying an attachment portion of the first sheet.

### BEST MODE FOR CARRYING OUT THE INVENTION

Hereinafter, one or more embodiments of the present invention will be described with reference to the drawings. In the following description, the same members are denoted by the same reference numerals and the description of members once explained may be omitted. In addition, each figure illustrates X-Y-Z coordinates as reference coordinates. In the following description, Z1-Z2 direction will be referred to as the up-down direction, X1-X2 direction will be referred to as the front-back direction, and Y1-Y2 direction will be referred to as the left-right direction. The X1-X2 direction and the Y1-Y2 direction are orthogonal to each other, and the X-Y plane including them is orthogonal to the Z1-Z2 direction. The state viewed from the upper side (Z1 side) to the lower side (Z2 side) may be referred to as a plan view.

### (Configuration of medical robot)

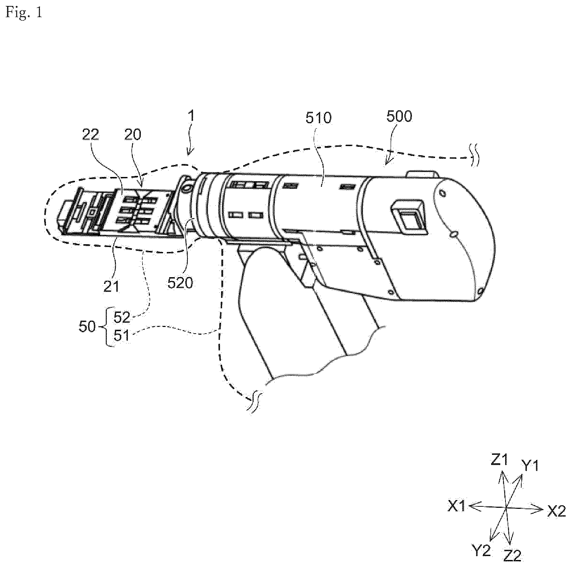

FIG. 1 is a perspective view for exemplifying the configuration of a medical robot.

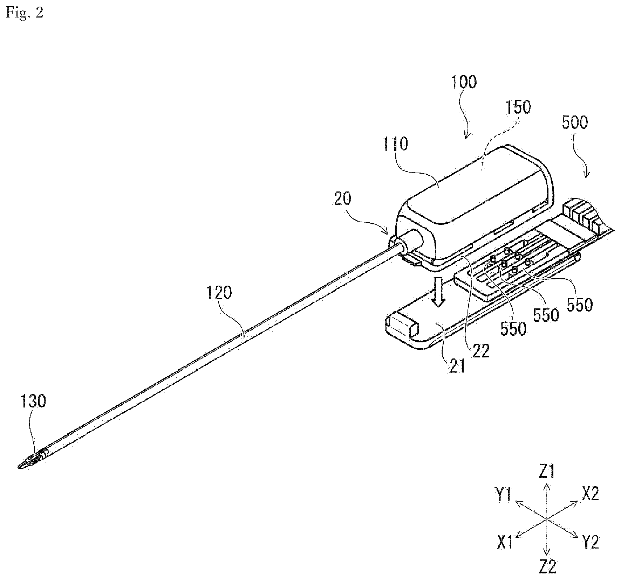

FIG. 2 is a perspective view for exemplifying the attaching state of an insertion member.

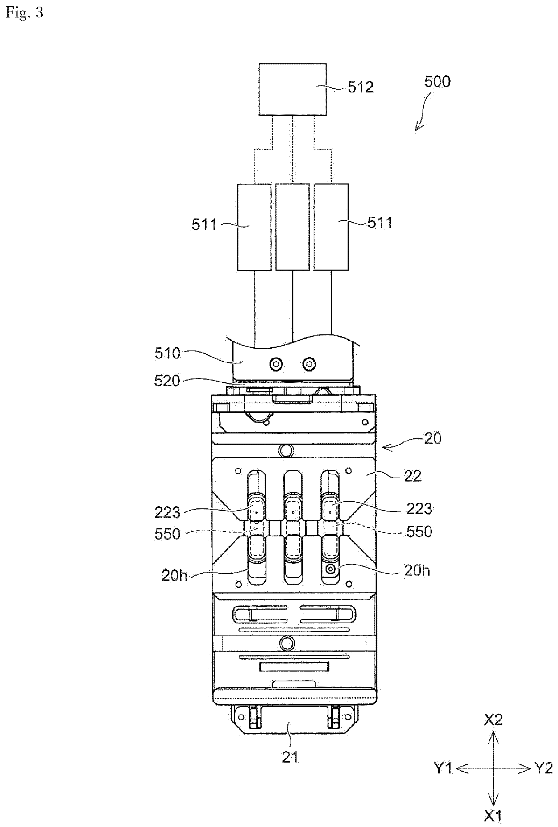

FIG. 3 is a plan view for exemplifying a driving portion of the medical robot.

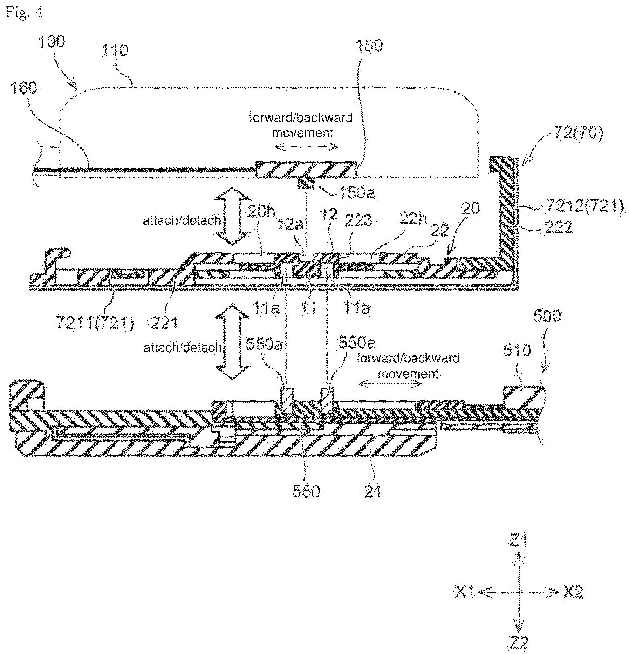

FIG. 4 is a cross-sectional view for exemplifying a connection configuration via a separator.

As illustrated in FIG. 1, a medical robot 500 has an arm portion 510 that is a remotely-operable manipulator whose direction and angle can be changed. The tip of the arm portion 510 is provided with a holder 21. From the front end of the holder 21, the holder 21 and a separator 22 of an attachment 20 extend forward. The attachment 20 is arranged to cover the holder 21. An insertion member 100 (see FIG. 2) is detachably attached with respect to the holder 21 by the separator 22. The insertion member 100 is inserted into the body of a patient who is an object of treatment with the medical robot 500. The posture of the insertion member 100 is changed in accordance with changes in the direction and angle of the arm portion 510. Examples of the insertion member 100 include a treatment instrument used for the treatment and an endoscope.

As illustrated in FIG. 2, the insertion member 100 includes a main body 110, a shaft 120 extending from the main body 110, and a treatment part 130 provided at the tip of the shaft 120 (an end portion opposite to the main body 110). The treatment part 130 is, for example, forceps. FIG. 2 illustrates a state in which the separator 22 of the attachment 20 is attached to the insertion member 100, but at the time of actual treatment, the separator 22 is first attached to the holder 21 of the medical robot 500 as illustrated in FIG. 1, and the insertion member 100 is then attached to the separator 22 which has been attached to the holder 21.

The insertion member 100 attached to the medical robot 500 is subjected to a sterilization treatment and placed in a clean area on the treatment room side. On the other hand, the medical robot 500 is placed in an unclean area that is not as clean as the insertion member 100. For this reason, during the treatment, the medical robot 500 is covered with a sheet 50 of a drape 1 (see the broken line of FIG. 1) to zone the clean area and the unclean area. The drape 1 is also subjected to a sterilization treatment.

Here, the drape 1 has the attachment 20 with the separator 22, and the sheet 50 having flexibility. The sheet is provided inseparably from the attachment 20. Details of the drape 1 will be described later.

As illustrated in FIG. 3, the medical robot 500 is provided with actuator parts 511 and a control unit 512. The actuator parts 511 generate driving force for operating the insertion member 100. The actuator parts 511 are connected to respective power transmission parts 550 that transmit driving force in the front-back direction (X1-X2 direction) to the attachment 20.

The present embodiment will be described as being applied to an example in which the actuator parts 511 generate driving force using a gas such as air or a fluid. The actuator parts 511 may use electric motors, and the type of generating the power is not limited. The actuator parts 511 may have a configuration using a piston and a cylinder or a configuration in which the driving force is generated from other known fluids, and the specific configuration is not limited.

The control unit 512 controls generation of the driving force in the actuator parts 511. The control unit 512 also controls the movement in the X direction in the power transmission parts 550 (FIG. 2) and the arrangement positions of the power transmission parts 550. The present embodiment will be described as being applied to an example in which the control unit 512 controls the supply of a gas such as air to the actuator parts 511.

To attach the insertion member 100 to the holder 21, the main body 110 of the insertion member 100 is fitted to the separator 22 attached to the holder 21 so as to be slid, for example, in the Y direction.

By fitting the main body 110 to the separator 22, the power from the power transmission parts 550 can be transmitted to the main body 110 via sliders 223 of the separator 22. Movable parts 150 are provided in the main body 110. The movable parts 150 are coupled with the treatment part 130 via wires 160 and can perform respective predetermined movements (e.g., movements in the front-back direction). Movements of the power transmission parts 550 (e.g., movements in the front-back direction) are transmitted from the sliders 223 of the separator 22 to the movable parts 150 of the insertion member 100 and can operate the treatment part 130 via the wires 160 (e.g., can open and close the forceps). That is, the separator 22 transmits the power in the forward and backward directions from the power transmission parts 550 of the medical robot 500 to the movable parts 150 provided in the insertion member 100.

As illustrated in FIG. 4, the separator 22 has a separator main body 221 and sliders 223. The separator main body 221 is provided with through-holes 20h through which respective parts of the sliders 223 are inserted, and is detachably attached with respect to the medical robot 500. The sliders 223 are provided so as to be slidable in the front-rear direction (X1-X2 direction) with respect to the separator main body 221. The sliders 223 have first engagement portions 11 that engage with the power transmission parts 550 and second engagement portions 12 that engage with the movable parts 150 of the insertion member 100, and transmit the power received from the power transmission parts 550 to the movable parts 150.

The first engagement portions 11 of the separator 22 are provided with recessed portions 11a into which protruding portions (power-side protruding portions 550a) of the power transmission parts 550 are fitted. When the separator 22 is attached to the holder 21 of the arm portion 510, the power-side protruding portions 550a of the power transmission parts 550 are fitted into the recessed portions 11a of the first engagement portions 11 of the sliders 223. This allows the power when the power transmission parts 550 move forward/backward to be transmitted to the first engagement portions 11, and the sliders 10 can achieve the front-back movements.

The second engagement portions 12 are provided with recessed portions 12a into which protruding portions (insertion-side protruding portions 150a) of the movable parts 150 of the insertion member 100 are fitted. When the main body 110 of the insertion member 100 is attached to the separator 22, the insertion-side protruding portions 150a protruding from the back surface of the main body 110 are fitted into the recessed portions 12a of the second engagement portions 12. This allows the sliders 223 and the movable parts 150 of the insertion member 100 to be engaged with each other, and the front-back movements of the power transmission parts 550 can be transmitted from the sliders 223 to the movable parts 150 of the insertion member 100. That is, when the power transmission parts 550 are moved in the front-back direction, the power is transmitted from the sliders 223 to the movable parts 150, and the movements of the movable parts 150 in the front-back direction can be transmitted to the wires 160 to operate the treatment part 130 via the wires 160.

### (Bearing structure of drape)

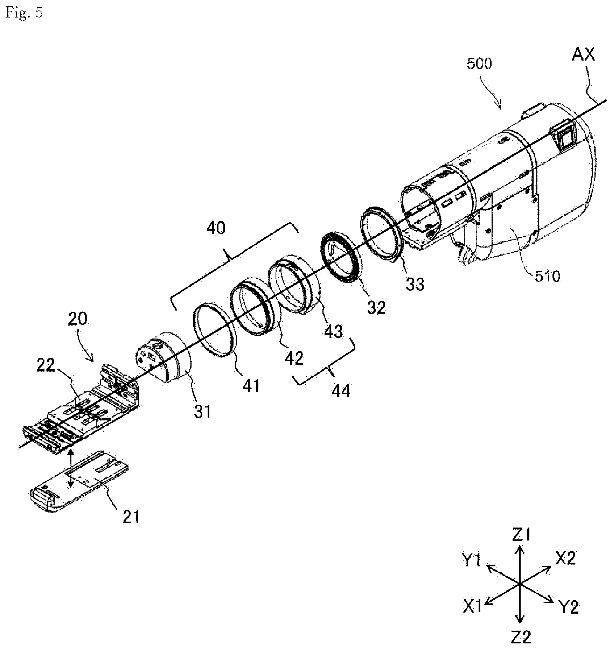

FIG. 5 is an exploded perspective view for exemplifying the schematic configuration of the arm tip side of the medical robot.

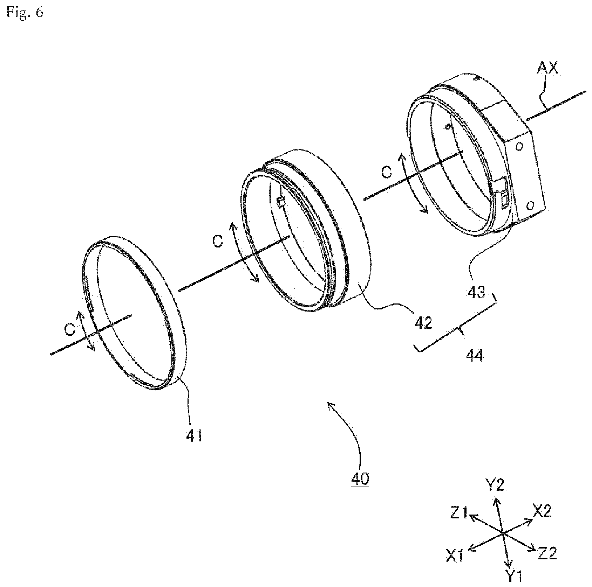

FIG. 6 is an exploded perspective view for exemplifying the configuration of a bearing structure.

As illustrated in FIG. 5, the holder 21 and the separator 22 are attached to the arm portion 510 via a switch unit 31, a bearing structure 40, and a bearing member 32 that are arranged in this order from the front to the rear. The bearing member 32 is fixed to the arm portion 510 via an annular bearing plate 33. The holder 21 is provided integrally with the switch unit 31. The separator 22 is detachably attached to the holder 21. The bearing member 32 is, for example, a rolling bearing whose outer ring is fixed to the arm portion 510 and whose inner ring can rotate relative to the outer ring.

As illustrated in FIGS. 5 and 6, the bearing structure 40 includes a rotating member 41, a joint member 42, and a base member 43 that are all ring-shaped. The joint member 42 serves as a fixation member. Rear portions of the holder 21 and the separator 22 are inserted inside the above members of the bearing structure 40, and the holder 21 and the separator 22 are provided integrally with the rotating member 41.

The joint member 42 and the base member 43 are detachably attached to each other by fitting and constitute a fixation member 44. The base member 43 is attached with respect to the arm portion 510. The rotating member 41 is attached with respect to the joint member 42 of the fixation member 44 in a slidable manner along a circumferential direction C.

The base member 43 is preferably composed of a metal from the viewpoint of durability against detachable attachment with the joint member 42, and for example, aluminum or duralumin is used.

The joint member 42 and the rotating member 41 are preferably composed of a resin material from the viewpoint of slidability and durability against sterilization, and for example, ABS resin, polycarbonate, and polyacetal are suitable.

By configuring the rotating member 41 and the fixation member 44 with such materials, it is possible to ensure the slidability for a long period of time, and damage due to sliding over a long period of time can be reduced.

The holder 21, the separator 22, the switch unit 31, and the rotating member 41 are supported by the bearing member 32 in a rotatable manner and are driven by a motor (not illustrated) to rotate around an axis of rotation AX along the front-rear direction (X1-X2 direction).

In the drape 1, the detachable attachment of the sheet 50 is performed via the attachment 20. The base member 43 is fixed to the arm portion 510 side, and members (rotating member 41 and separator 22) on the tip side of the joint member 42 can be detachably attached with respect to the base member 43. In the present embodiment, therefore, the attachment 20 may be configured to include the attachment 20, the separator 22, the rotating member 41, and the joint member 42.

### (Configuration of drape)

Details of the drape 1 will then be described.

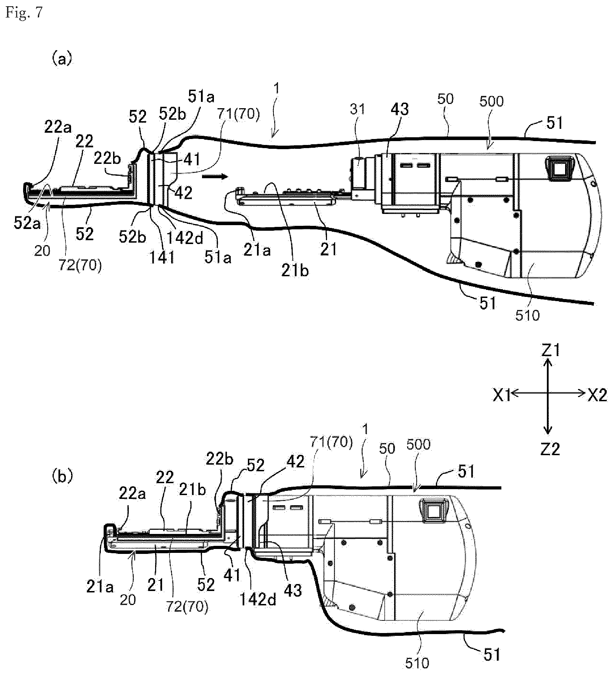

FIGS. 7(a) and 7(b) are diagrams for exemplifying the structure of the drape.

FIG. 7(a) is a side view illustrating a state before the drape 1 is attached, and FIG. 7(b) is a side view illustrating a state in which the drape 1 is attached to the holder 21 side and the arm portion 510 side.

As illustrated in FIGS. 7(a) and 7(b), during the treatment, the medical robot 500 is covered with the drape 1 having an approximately tubular shape with a plurality of opening portions. Even when the medical robot 500 is covered with the drape 1, the insertion member 100 can be detachably attached to the holder 21, and the holder 21 to which the insertion member 100 is attached can be rotated.

The drape 1 according to the present embodiment includes the attachment 20, the sheet 50, and a pinch prevention unit 70. The attachment 20 has the separator 22 described previously. That is, the separator 22 is disposed between the medical robot 500 and the insertion member 100 to isolate the insertion member 100 and the medical robot 500 from each other, and serves also to transmit the power in the front-back direction from the power transmission parts 550 of the medical robot 500 to the movable parts 150 (see FIG. 2) provided in the insertion member 100.

The sheet 50 has a first sheet 51 fixed to the joint member 42 of the bearing structure 40 and a second sheet 52 fixed along the rotating member 41 of the bearing structure 40.

Specifically, a first opening portion 51a that is a front opening portion (on the X1 side in the X1-X2 direction) of the tubular first sheet 51 is fixed along an outer peripheral surface 142d on the rear side of the joint member 42. The first sheet 51 extends rearward from the joint member 42 and covers the surgical robot including the arm portion 510.

Here, the fixation member 44 (see FIG. 5) is composed of two components of the base member 43 fixed to the arm portion 510 and the joint member 42 that can be detachably attached to the base member 43, and it is therefore easy to detachably attach the first sheet 51 with respect to the arm portion 510.

A front opening portion 52a of the tubular second sheet 52 is provided in a linked manner with the separator 22 so as to be fixed from its front end portion 22a to its rear end portion 22b. This allows the separator 22 and the second sheet 52 to be integrated, and the lower surface of the separator 22 is placed in the second sheet 52.

On the other hand, a rear opening portion 52b of the second sheet 52 is fixed along an outer peripheral surface 141 of the rotating member 41.

Thus, the separator 22 and the rotating member 41 are attached with respect to the arm portion 510 from the state in which the second sheet 52 is attached. In this operation, the holder 21 and the switch unit 31 are inserted into the rotating member 41, and the separator 22 is attached to an upper surface 21b of the holder 21. According to this step, as illustrated in FIG. 7(b), the front surface and lower surface of the holder 21 are covered with the second sheet 52 from a front end portion 21a located on the front side of the front end portion 22a of the separator 22, and the upper surface 21b is covered with the separator 22. When the rotating member 41 rotates relative to the fixation member 44, the second sheet 52 rotates integrally with the rotating member 41, the separator 22, and the holder 21.

### (Pinch prevention unit)

The pinch prevention unit 70 is provided inseparably from the attachment 20 and has flexibility. As described above, when the medical robot 500 is covered with the drape 1, the sheet 50 having flexibility is likely to be pinched between the attachment 20 and the connection portion. The pinch prevention unit 70 according to the present embodiment effectively prevents the sheet 50 having flexibility from being pinched between the attachment 20 and the connection portion when covering the medical robot 500 with the drape 1.

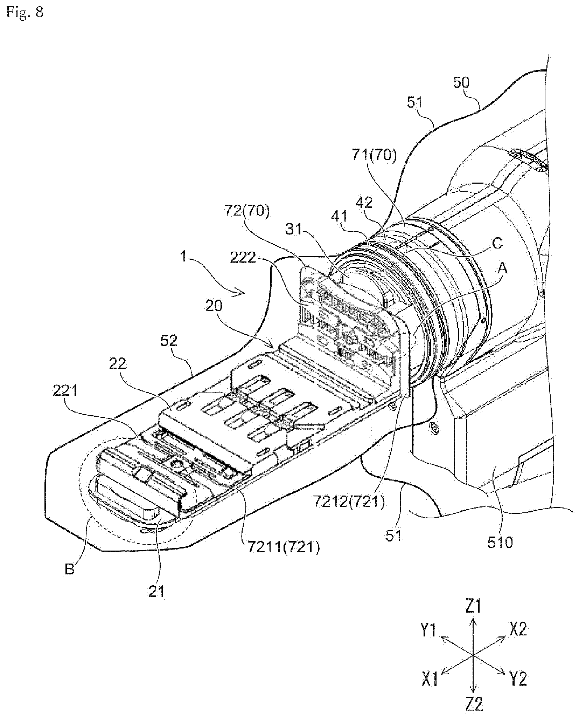

FIG. 8 is a perspective view for exemplifying the pinch prevention unit.

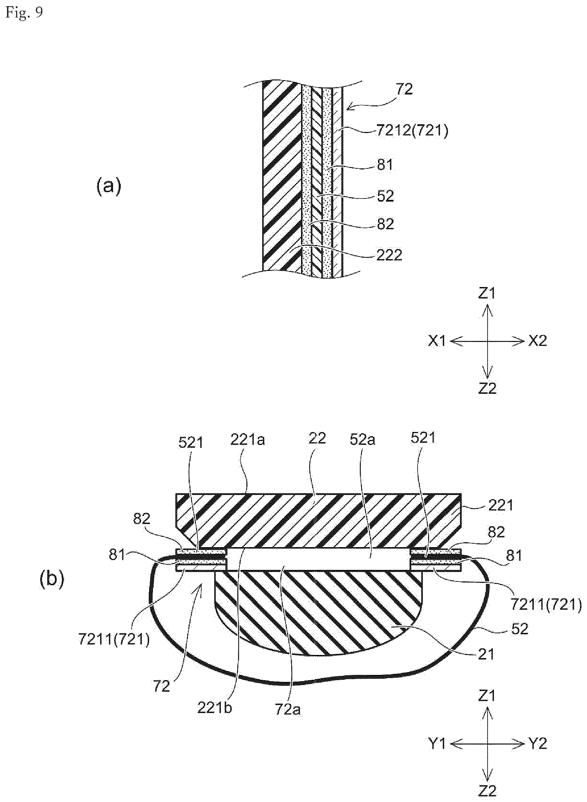

FIGS. 9(a) and 9(b) are schematic cross-sectional views for exemplifying the pinch prevention unit. FIG. 9(a) illustrates a cross-sectional view of section A of FIG. 8 viewed in the Y1 direction, and FIG. 9(b) illustrates a cross-sectional view of section B of FIG. 8 viewed in the X2 direction.

The pinch prevention unit 70 is provided at a position corresponding to the connection portion between the arm portion 510 and the drape 1. In the present embodiment, the joint member 42 to which the first sheet 51 is connected is provided with a first pinch prevention part 71. In addition, in the present embodiment, the separator 22 to which the second sheet 52 is connected is provided with a second pinch prevention part 72.

This will be more specifically described. When the first sheet 51 is placed over the arm portion 510, the joint member 42 to which the first sheet 51 is connected is fitted to the base member 43. In this operation, the first pinch prevention part 71 prevents the first sheet 51 from being pinched between the joint member 42 and the base member 43. In addition, when the second sheet 52 is placed over the holder 21 and the separator 22 is attached to the holder 21, the second pinch prevention part 72 prevents the second sheet 52 from being pinched between the separator 22 and the holder 21 and also prevents the second sheet 52 from being pinched between the separator 22 and the switch unit 31.

While it is desired to provide both the first pinch prevention part 71 and the second pinch prevention part 72, either one of them may be provided.

### (Second pinch prevention part)

The second pinch prevention part 72 includes a plate-shaped body 721 having an extending portion that extends outward from the outer surface of the separator 22. For example, the separator 22 has a separator main body 221 that comes into contact with the holder 21, and an upright portion 222 that is provided to stand up from the separator main body 221 on its rear end side. The second pinch prevention part 72 has a first plate-shaped body 7211 that extends outward from the separator main body 221, and a second plate-shaped body 7212 that extends outward from the upright portion 222.

The material of the plate-shaped body 721 is, for example, polyethylene terephthalate (PET). A part of the plate-shaped body 721 having a flat plate shape is folded thereby to configure the first plate-shaped body 7211 facing the separator main body 221 and the second plate-shaped body 7212 facing the upright portion 222. The flexibility of the second pinch prevention part 72 is higher than that of the attachment 20 and lower than that of the sheet 50 (second sheet 52). Thus, the first plate-shaped body 7211 extends outward from the edge of the separator main body 221, and the second plate-shaped body 7212 extends outward from the edge of the upright portion 222, so that extending portions of the first plate-shaped body 7211 and second plate-shaped body 7212 serve as wing-shaped supports to prevent the second sheet 52 from approaching the separator 22.

Therefore, when the separator 22 is attached to the holder 21 in a state in which the sheet 50 is placed over the medical robot 500, the soft second sheet 52 can be prevented from approaching the edge of the separator 22 and can also be prevented from being pinched between the separator 22 and the holder 21 or between the separator 22 and the switch unit 31.

That is, when the separator 22 is attached to the holder 21, the first plate-shaped body 7211 prevents the second sheet 52 from approaching the edge of the separator main body 221 and also prevents the second sheet 52 from being pinched between the separator main body 221 and the holder 21.

Additionally or alternatively, when the separator 22 is coupled with the switch unit 31, the second plate-shaped body 7212 prevents the second sheet 52 from approaching the edge of the upright portion 222 and also prevents the second sheet 52 from being pinched between the upright portion 222 and the switch unit 31. Furthermore, in the state in which the separator 22 is coupled with the switch unit 31, the second plate-shaped body 7212 extends in the direction away from the rotating member 41, so when the holder 21 and the attachment 20 rotate, it is possible to prevent the jamming of the second sheet 52 in an area around the rotating member 41.

Preferably, at least the extending portion of the plate-shaped body 721 of the second pinch prevention portion 72 has translucency. According to this feature, when the separator 22 is attached to the holder 21, it is easy to refer to the attachment situation through the second pinch prevention part 72 having translucency.

As illustrated in FIGS. 9(a) and 9(b), the plate-shaped body 721 is inseparably attached to the second sheet 52 in an area including the end portion of the extending portion. In the present embodiment, the plate-shaped body 721 is attached to the second sheet 52 via a first adhesive layer 81. The first adhesive layer 81 is, for example, a double-sided tape. The first adhesive layer 81 is provided on at least a part of the outer surface of the plate-shaped body 721 of the second pinch prevention portion 72. Thus, the second sheet 52 and the second pinch prevention part 72 are inseparably attached to each other by the first adhesive layer 81, and it is thereby possible to reliably isolate the clean area and the unclean area between the second sheet 52 and the second pinch prevention part 72.

Here, the first adhesive layer 81 is preferably provided on one main surface of the plate-shaped body 721 and preferably extends to the inner end portion of the extending portion of the plate-shaped body 721. This allows the second sheet 52 and the plate-shaped body 721 to be attached to each other by the first adhesive layer 81 up to the end portion of the extending portion, and the clean area and the unclean area can be separated more reliably.

In addition, a second adhesive layer 82 is provided so as to cover at least a part of the portion of the second sheet 52 that is attached to the first adhesive layer 81. That is, the second adhesive layer 82 is provided on the side of the second sheet 52 opposite to the side on which the first adhesive layer 81 is provided. The second adhesive layer 82 is, for example, a double-sided tape. This allows the second sheet 52 to have an interposed portion 521 (see FIG. 9(b)) interposed between the first adhesive layer 81 and the second adhesive layer 82. The second sheet 52 is attached to the separator 22 of the attachment 20 by the second adhesive layer 82 at the interposed portion 521.

In other words, the separator 22 of the attachment 20 is adhered to the second adhesive layer 82 on the side opposite to the side facing the second sheet 52. This allows the second sheet 52 to be interposed between the first adhesive layer 81 and the second adhesive layer 82 by the interposed portion 521, so that the clean area and the unclean area can be reliably isolated.

This interposed portion 521 may include a folded portion of the second sheet 52. The second sheet 52 has a folded portion extending from the separator main body 221 to the upright portion 222 of the separator 22. Preferably, the interposed portion 521 is also provided at a folded portion of the second sheet 52 that faces the upright portion 222. This allows the second sheet 52 including its folded portion to be interposed between the first adhesive layer 81 and the second adhesive layer 82, and the clean area and the unclean area are reliably isolated.

As illustrated in FIG. 9(b), preferably, the portion of the plate-shaped body 721 located around the sliders 223 of the separator 22 (see FIG. 4) does not have a portion that protrudes further in a direction in the main surface of the plate-shaped body 721 than the separator main body 221 when the portion is viewed from the normal direction (Z1-Z2 direction, Z2 direction).

That is, a lower surface 221b of the separator main body 221 (the surface that contacts the holder 21) is provided to be narrower than an upper surface 221a of the separator main body 221 (the surface that is opposite to the surface that contacts the holder 21). The plate-shaped body 721 extends outward beyond the edge of the lower surface 221b of the separator main body 221, but does not extend outward beyond the edge of the upper surface 221a of the separator main body 221. Therefore, when the separator 22 is viewed in the Z2 direction of the Z1-Z2 direction, the plate-shaped body 721 is hidden behind the separator main body 221 and cannot be seen. On the other hand, when the separator 22 is viewed in the Z1 direction of the Z1-Z2 direction, the plate-shaped body 721 is in a state of extending outward from the lower surface 221b of the separator main body 221. It is thereby possible to obtain the effect of preventing the second sheet 52 from being pinched without extending the plate-shaped body 721 more than necessary.

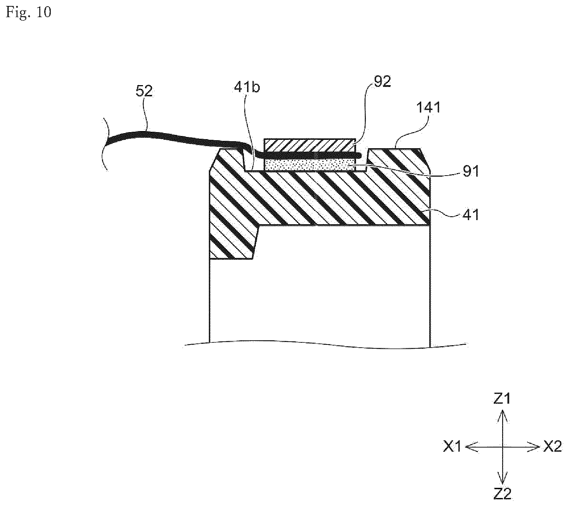

FIG. 10 is a schematic cross-sectional view for exemplifying a connection portion between the second sheet and the rotating member. FIG. 10 illustrates a cross-sectional view of section C of FIG. 8 viewed in the Y1 direction.

As illustrated in FIG. 10, the end portion of the second sheet 52 is connected to a recessed portion 41b provided in the outer peripheral surface 141 of the rotating member 41 of the bearing structure 40 with a double-sided tape 91. The recessed portion 41b is provided along the entire circumferential direction of the rotating member 41. Therefore, the end portion of the second sheet 52 is connected to the entire circumferential direction of the recessed portion 41b of the rotating member 41 with the double-sided tape 91. In addition, the end portion of the second sheet 52 is fixed from above with a tape 92 for pressing. This allows the second sheet 52 and the rotating member 41 to be connected without a gap, and the clean area and the unclean area can be reliably isolated.

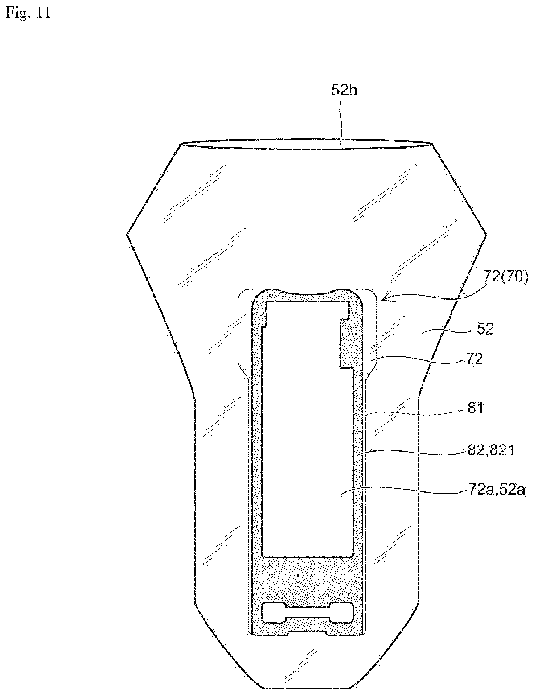

FIG. 11 is a plan view for exemplifying an opening of the second sheet and the pinch prevention unit.

FIG. 11 illustrates a state before the separator 22 is attached to the second sheet 52.

The front opening portion 52a is provided in the central portion on the front surface side of the bag-shaped second sheet 52, and the separator 22 is attached to cover the front opening portion 52a. The second pinch prevention part 72 has the plate-shaped body 721 having a shape that extends outward from the edge of the front opening portion 52a. The second pinch prevention part 72 is attached to the inside of the bag of the second sheet 52. That is, the second pinch prevention part 72 has an opening portion 72a and is attached to the inside of the bag of the second sheet 52 by the first adhesive layer 81 so that the opening portion 72a and the front opening portion 52a are aligned. This allows the second sheet 52 and the second pinch prevention part 72 to be connected so that the second sheet 52 does not cover the opening portion 72a of the second pinch prevention part 72. In addition, the second adhesive layer 82 is attached to the outside of the bag of the second sheet 52 so as to rim the front opening portion 52a of the second sheet 52. This second adhesive layer 82 allows the separator 22 to be attached over the front opening portion 52a of the second sheet 52.

In a state before the separator 22 is attached by the second adhesive layer 82, a cover film 821 that protects the second adhesive layer 82 is preferably provided on the side of the second adhesive layer 82 opposite to the side facing the second sheet 52. This can prevent the second adhesive layer 82 from attaching to an unintended site at a stage before the separator 22 and the second sheet 52 are adhered together.

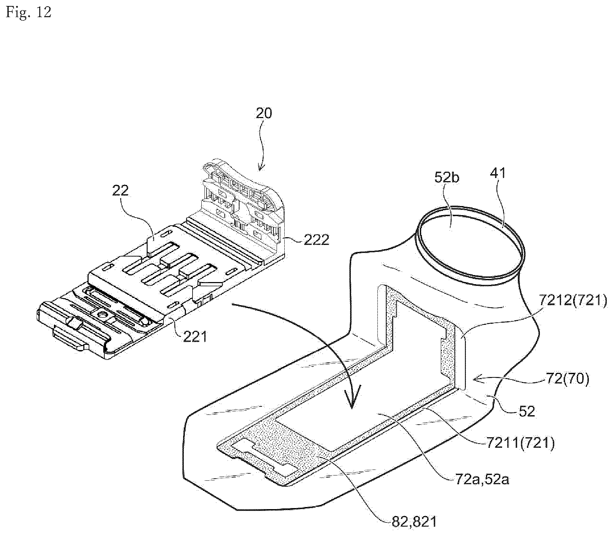

FIG. 12 is a perspective view for exemplifying a state of the second sheet and the separator before adhesion. To adhere the separator 22 to the second sheet 52, the second pinch prevention part 72 is attached to the inside of the bag of the second sheet 52, and in this state a part of the rear end side of the second pinch prevention part 72 is folded approximately vertically. The second pinch prevention part 72 is formed of PET, so the folded shape of the second pinch prevention part 72 is maintained due to the fold habit of PET. This folded portion faces the upright portion 222 of the separator 22.

In this state, the cover film 821 of the second adhesive layer 82 is removed, and the separator 22 is attached to the second sheet 52 using the second adhesive layer 82. This allows the separator 22 to be attached to the position of the front opening portion 52a of the second sheet 52.

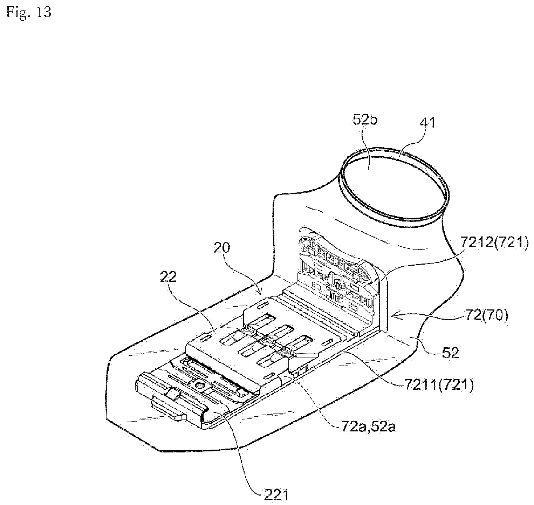

FIG. 13 is a perspective view for exemplifying a state of the second sheet and the separator after adhesion.

In a state in which the separator 22 is attached to the second sheet 52, the second sheet 52 having flexibility is supported by the second pinch prevention part 72, and is less likely to get pinched on the back side of the separator 22.

The ring-shaped rotating member 41 is attached to the rear opening portion 52b of the second sheet 52. At the position of the front opening portion 52a on the front surface side of the bag-shaped second sheet 52, the separator 22 is disposed between the inside and outside of the bag, and the operation from the medical robot 500 can be transmitted from the inside of the bag to the outside of the bag via the separator 22.

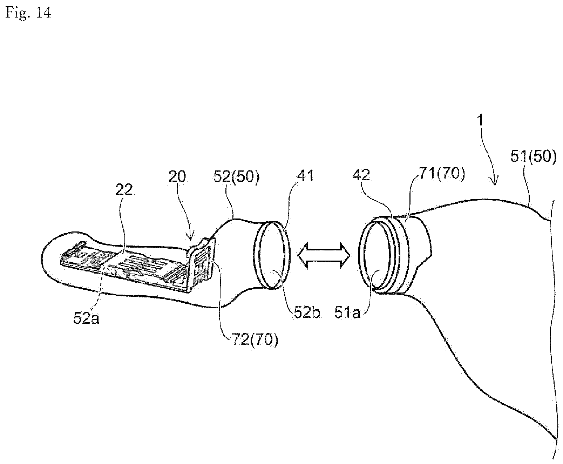

FIG. 14 is a schematic diagram for exemplifying the coupling between the first sheet and the second sheet.

The rotating member 41 attached to the rear opening portion 52b of the second sheet 52 is coupled with the joint member 42 of the first sheet 51. As described previously, the rotating member 41 is rotatably coupled with the joint member 42. This allows the second sheet 52 side to be rotatable even though the first sheet 51 and the second sheet 52 are integrated. When the holder 21 portion provided at the tip of the arm portion 510 of the medical robot 500 rotates, the second sheet 52 also rotates together with the separator 22, and the occurrence of twisting of the drape 1 can be prevented.

### (First pinch prevention part)

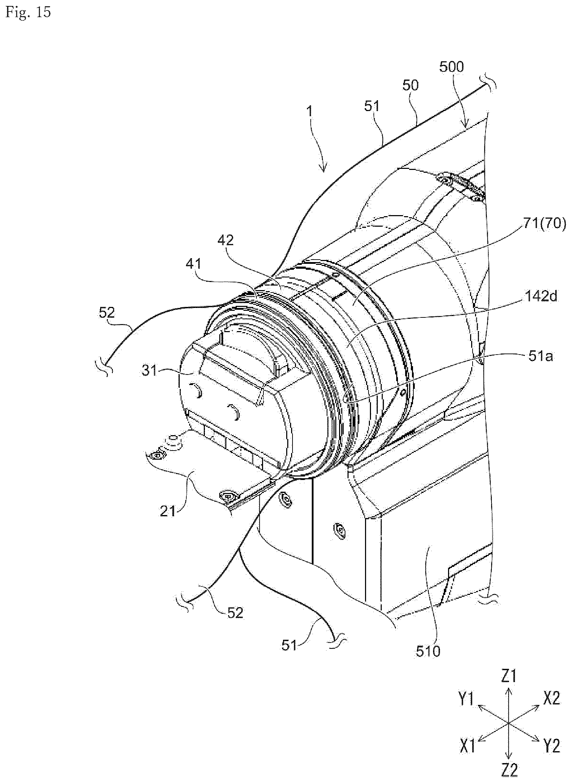

FIG. 15 is a perspective view for exemplifying an attachment portion of the first sheet.

The first opening portion 51a of the first sheet 51 is fixed along the outer peripheral surface 142d of the joint member 42. The first pinch prevention part 71 is provided between the first sheet 51 and the outer peripheral surface 142d of the joint member 42.

The first pinch prevention part 71 is provided along the outer peripheral surface 142d of the joint member 42, and is provided so as to extend rearward (in an approximately tubular shape). With this configuration, when the first sheet 51 is placed over the holder 21 provided at the tip of the arm portion 510 of the medical robot 500, the first pinch prevention part 71 can prevent jamming of the first sheet 51 on the outer periphery of the joint member 42.

That is, the first sheet 51 is provided in a size that covers the arm portion 510, so it is provided in a large bag shape rearward from the joint member 42. The joint member 42 is fitted to the base member 43 in a state in which the bag-shaped first sheet 51 is placed over the arm portion 510. In this operation, the first sheet 51 is likely to be jammed between the joint member 42 and the base member 43. In the present embodiment, since the joint member 42 is provided with the first pinch prevention part 71, the space between the joint member 42 and the base member 43 is covered with the first pinch prevention part 71, and the first sheet 51 can be prevented from being pinched between the joint member 42 and the base member 43.

Here, the flexibility of the first pinch prevention part 71 is higher than that of the joint member 42 and lower than that of the sheet 50 (first sheet 51). This allows the extending portion of the first pinch prevention part 71 to serve as a support and prevent the first sheet 51 from approaching the joint member 42. Therefore, when the joint member 42 is fitted to the base member 43 in a state in which the sheet 50 is placed over the medical robot 500, the soft first sheet 51 can be prevented from approaching the edge of the joint member 42 and can also be prevented from being pinched between the joint member 42 and the base member 43.

Preferably, at least the extending portion of the first pinch prevention part 71 has translucency. According to this feature, when the joint member 42 is fitted to the base member 43, it is easy to refer to the fitting situation through the first pinch prevention part 71 having translucency.

Thus, according to the present embodiment, the clean area and the unclean area can be sufficiently isolated, and when the drape 1 is attached to the medical robot 500, the sheet 50 of the drape 1 can be avoided from being pinched, and it is possible to prevent poor fixation of the attachment 20 and damage to the drape 1.

Although the present embodiments have been described above, the present invention is not limited to these examples. For example, the scope of the present invention encompasses those to which a person skilled in the art appropriately makes addition or removal of constitutional elements or design changes with respect to the previously-described embodiments and those in which features of the embodiments are appropriately combined, provided that they have the subject matters of the present invention as defined in the appended claims. For example, the insertion member 100 may be a part of an endoscope.

### DESCRIPTION OF REFERENCE NUMERALS

- 1: Drape
- 11: First engagement portion
- 11a: Recessed portion
- 12: Second engagement portion
- 12a: Recessed portion
- 20: Attachment
- 20h: Through-hole
- 21: Holder
- 21a: Front end portion
- 21b: Upper surface
- 22: Separator
- 22a: Front end portion
- 22b: Rear end portion
- 31: Switch unit
- 32: Bearing member
- 33: Bearing plate
- 40: Bearing structure
- 41: Rotating member
- 41b: Recessed portion
- 42: Joint member
- 43: Base member
- 44: Fixation member
- 50: Sheet
- 51: First sheet
- 51a: First opening portion
- 52: Second sheet
- 52a: Front opening portion
- 52b: Rear opening portion
- 70: Pinch prevention unit
- 71: First pinch prevention part
- 72: Second pinch prevention part
- 72a: Opening portion
- 81: First adhesive layer
- 82: Second adhesive layer
- 91: Double-sided tape
- 92: Tape
- 100: Insertion member
- 110: Main body
- 120: Shaft
- 130: Treatment part
- 141: Outer peripheral surface
- 142d: Outer peripheral surface
- 150: Movable part
- 150a: Insertion-side protruding portion
- 160: Wire
- 221: Separator main body
- 221a: Upper surface
- 221b: Lower surface
- 222: Upright portion
- 223: Slider
- 500: Medical robot
- 510: Arm portion
- 511: Actuator part
- 512: Control unit
- 521: Interposed portion
- 550: Power transmission part
- 550a: Power-side protruding portion
- 721: Plate-shaped body
- 821: Cover film
- 7211: First plate-shaped body
- 7212: Second plate-shaped body
- AX: Axis of rotation
- C: Circumferential direction

## Claims

1. A drape (1) for covering a medical robot (500), comprising:
an attachment (20) for attachment to a connection portion of the medical robot (500);
a sheet (50) having flexibility that is provided inseparably from the attachment (20) and for covering the medical robot (500); and
a pinch prevention unit (70) that is provided on the attachment (20) and for preventing the sheet (50) from being pinched between the attachment (20) and the connection portion,
wherein a first adhesive layer is provided on at least a part of an outer surface of the pinch prevention unit (70), and the sheet (50) and the pinch prevention unit (70) are inseparably attached to each other by the first adhesive layer (81),
**characterized in in that**
the drape (1) has a second adhesive layer (82) provided on a side of the sheet (50) opposite to a side to which the first adhesive layer (81) is attached, and the sheet (50) has an interposed portion (521) that is interposed between the first adhesive layer (81) and the second adhesive layer (82), and
the attachment (20) is located on a side of the second adhesive layer (82) opposite to a side facing the sheet (50).

2. The drape (1) according to claim 1, wherein the flexibility of the pinch prevention unit (70) is higher than that of the attachment (20) and lower than that of the sheet (50).

3. The drape (1) according to claim 1 or 2, wherein the pinch prevention unit (70) has translucency.

4. The drape (1) according to any one of claims 1 to 3, wherein the pinch prevention unit (70) includes a plate-shaped body (721) having an extending portion that extends outward from an outer surface of the attachment (20), and the first adhesive layer (81) is provided on one main surface of the plate-shaped body (721) and extends to an end portion of the extending portion of the plate-shaped body (721).

5. The drape (1) according to any one of claims 1 to 4, wherein the interposed portion (521) includes a folded portion of the sheet (50).

## Patentansprüche

1. Drape (1) zur Abdeckung eines medizinischen Roboters (500), enthaltend:
eine Befestigungseinheit (20) zur Befestigung an einem Verbindungsabschnitt des medizinischen Roboters (500);
eine Folie (50) mit Flexibilität, die untrennbar mit der Befestigungseinheit (20) und zur Abdeckung des medizinischen Roboters (500) bereitgestellt ist; und
eine Einklemmverhinderungseinheit (70), die an der Befestigungseinheit (20) und zum Verhindern bereitgestellt ist, dass die Folie (50) zwischen der Befestigungseinheit (20) und dem Verbindungsabschnitt eingeklemmt wird,
wobei eine erste Klebstoffschicht an mindestens einem Teil einer äußeren Oberfläche der Einklemmverhinderungseinheit (70) bereitgestellt ist und die Folie (50) und die Einklemmverhinderungseinheit (70) durch die erste Klebstoffschicht (81) untrennbar aneinander befestigt sind,
**dadurch gekennzeichnet, dass**
das Drape (1) eine zweite Klebstoffschicht (82) aufweist, die an einer Seite der Folie (50) gegenüber einer Seite bereitgestellt ist, an der die erste Klebstoffschicht (81) befestigt ist, und die Folie (50) einen dazwischenliegenden Abschnitt (521) aufweist, der zwischen der ersten und der zweiten Klebstoffschicht (82) liegt, und
die Befestigungseinheit (20) an einer Seite der zweiten Klebstoffschicht (82) gegenüber einer Seite positioniert ist, die zur Folie (50) gerichtet ist.

2. Drape (1) gemäß Anspruch 1, wobei die Flexibilität der Einklemmverhinderungseinheit (70) höher ist als die der Befestigungseinheit (20) und niedriger als die der Folie (50).

3. Drape (1) gemäß Anspruch 1 oder 2, wobei die Einklemmverhinderungseinheit (70) Transluzenz aufweist.

4. Drape (1) gemäß einem der Ansprüche 1 bis 3, wobei die Einklemmverhinderungseinheit (70) einen plattenförmigen Körper (721) mit einem Erstreckungsabschnitt einschließt, der sich von einer äußeren Oberfläche der Befestigungseinheit (20) erstreckt, und die erste Klebstoffschicht (81) auf einer Hauptfläche des plattenförmigen Körpers (721) bereitgestellt ist und sich bis zu einem Endabschnitt des Erstreckungsabschnitts des plattenförmigen Körpers (721) erstreckt.

5. Drape (1) gemäß einem der Ansprüche 1 bis 4, wobei der dazwischenliegende Abschnitt (521) einen gefalteten Abschnitt der Folie (50) enthält.

## Revendications

1. Un drapé (1) pour couvrir un robot médical (500), comprenant :
un attachement (20) pour une connexion à une partie de connexion du robot médical (500) ;
une feuille (50) avec une flexibilité, laquelle est prévue indissociablement de l'attachement (20) et pour couvrir le robot médical (500) ; et
une unité de prévention des pincements (70), laquelle est prévue sur l'attachement (20) et pour prévenir la feuille (50) d'être pincée entre l'attachement (20) et la partie de connexion,
dans lequel une première couche adhésif est prévue sur au moins une partie d'une surface extérieure de l'unité de prévention des pincements (70), et la feuille (50) et l'unité de prévention des pincements (70) sont indissociablement attachées l'une à l'autre par la première couche adhésive (81),
**caractérisé en ce que**
le drapé (1) a une seconde couche adhésive (82) prévue sur un côté de la feuille (50) opposé à un côté auquel la première couche adhésive (81) est attachée, et la feuille (50) a une portion interposée (521) qui est interposée entre la première couche adhésive (81) et la seconde couche adhésive (82), et
l'attachement (20) est située sur un côté de la seconde couche adhésive (82) opposé à un côté faisant face à la feuille (50).

2. Le drapé (1) selon la revendication 1, dans lequel la flexibilité de l'unité de prévention des pincements (70) est supérieure à celle de l'attachement (20) et inférieure à celle du drapé (50).

3. Le drapé (1) selon la revendication 1 ou 2, dans lequel l'unité de prévention des pincements (70) a une translucidité.

4. Le drapé (1) selon l'une des revendications 1 à 3, dans lequel l'unité de prévention des pincements (70) comprend un corps en forme de plaque (721) avec une portion allongée s'étendant vers l'extérieur à partir d'une surface extérieure de l'attachement (20), et la première couche adhésif (81) est prévue sur une surface principale du corps en forme de plaque (721) et s'étend jusqu'à une partie terminale de la portion allongée du corps en forme de plaque (721).

5. Le drapé (1) selon l'une des revendications 1 à 4, dans lequel la portion interposée (521) inclut une portion pliée de la feuille (50).
